# EUROPÄISCHE PATENTSCHRIFT

(11) **EP 0 652 740 B1**
(45) Veröffentlichungstag und Bekanntmachung des Hinweises auf die Patenterteilung: **24.04.1996**
(21) Anmeldenummer: 93917591.5
(22) Anmeldetag: 17.07.1993
(51) Int. Cl.: A61K 7/06, C08G 63/60, C08G 73/16

(54) **VERWENDUNG VON POLYKONDENSATEN UND NEUE POLYKONDENSATE**
USE OF POLYCONDENSATES AND NEW POLYCONDENSATES
UTILISATION DE POLYCONDENSATS ET NOUVEAUX POLYCONDENSATS

(30) Priorität: 27.07.1992 DE 4224761
(43) Veröffentlichungstag der Anmeldung: 17.05.1995
(73) Patentinhaber: BASF Aktiengesellschaft, D-67063 Ludwigshafen (DE)
(72) Erfinder: NGUYEN KIM, Son, D-6944 Hemsbach (DE); SANNER, Axel, D-6710 Frankenthal (DE); SPERLING-VEITMEIER, Karin, D-6730 Neustadt (DE)
(86) Internationale Anmeldenummer: EP9301887
(87) Internationale Veröffentlichungsnummer: WO9402110

(56) Entgegenhaltungen:
- EP-A- 0 159 283
- DE-A- 1 495 100
- FR-A- 2 358 878

## Beschreibung

In der Kosmetik werden Haarbehandlungsmittel, die beispielsweise als Haarverfestiger oder Haarspray vorliegen, zum Festigen, Strukturverbessern und Formgeben der Haare verwendet. Die Haarbehandlungsmittel bestehen vorwiegend aus einer Lösung von filmbildenden Harzen oder synthetischen Polymeren. Bisher wurden in Haarbehandlungsmitteln hauptsächlich folgende Filmbildner verwendet: Schellack, Homo- und Copolymerisate des N-Vinylpyrrolidons, Copolymerisate von Vinylethern/Maleinsäurehalbestern, von (Meth)acrylsäure oder deren Estern und Amiden und Crotonsäure mit Vinylestern. Als Lösungsmittel wird hauptsächlich Ethanol verwendet. Die Polymerlösung wird durch Sprühen auf die Haare gebracht. Nach dem Trocknen des Lösungsmittels werden die Haare durch die Polymeren in der gewünschten Form fixiert. Die Polymeren sollten einerseits so hydrophil sein, daß sie leicht aus dem Haar ausgewaschen werden können, andererseits sollen sie hydrophob sein, damit die mit Polymeren behandelten Haare auch bei hoher Luftfeuchtigkeit in Form bleiben und nicht miteinander verkleben.

Die bisher bekannten polymeren Filmbildner, wie Polyvinylpyrrolidone zeigen jedoch meistens als Nachteil eine zu hohe Wasseraufnahme bei erhöhter Luftfeuchtigkeit. Diese Eigenschaft führt, wie schon gesagt, zu einem unerwünschten Verkleben der Haare und zu einem Verlust der Festigkeit und damit einem Zusammenbruch der Frisur. Wird andererseits die Widerstandsfähigkeit gegen hohe Luftfeuchtigkeit verbessert, z.B. bei Copolymerisaten aus N-Vinylpyrrolidon und Vinylacetat, so leidet darunter die Elastizität des Films und die Sprödigkeit dieser Filme kann nach der Haarbehandlung sogar zu einem unangenehmen Stauben und einem schuppigen Belag führen. Außerdem wird vor allem die Auswaschbarkeit sehr erschwert. Zudem sind die Polymeren wegen ihrer hydrolysebeständigen C-C-Ketten biologisch nicht abbaubar.

Schellack ist dagegen biologisch abbaubar, hat aber viele Nachteile. So sind seine Eigenschaften als Haarbehandlungsmittel im Vergleich zu den Homo- und Copolymerisaten des N-Vinylpyrrolidons schlechter, insbesondere bezüglich der Klebrigkeit, Wasserlöslichkeit und Steifigkeit. Da Schellack ein Naturprodukt ist, sind seine Eigenschaften außerdem starken Schwankungen unterlegen.

Aus den Schutzrechten US 4,300,580; US 3,734,874;
DE 26 33 418 B2; WO 89/07118 sind NaSO₃-Gruppen enthaltende Polyester bekannt, deren Hauptkette durch Kondensationsreaktion aufgebaut ist und bei denen zu erwarten ist, daß sie durch Hydrolyse der Estergruppierungen zu kürzeren Segmenten abgebaut werden können. Ein Nachteil dieser NaSO₃-haltigen Polyester ist aber die schlechte Ethanolverträglichkeit, die dazu führt, daß man die Polyester nur in Wasser oder Wasser/Ethanol-Gemischen verwenden kann, die natürlich nur schlecht trocknen.

Wasserlösliche oder in Wasser dispergierbare Polymere, z.B. Polyester, Polyamide oder Polyurethane gewinnen wegen ihrer durch geeignete Einsatzstoffe leicht einstellbaren Produkt-Eigenschaften immer mehr an Bedeutung. Es ist bekannt, daß Maleinsäureanhydrid und Trimellitsäureanhydrid zur Herstellung von H₂O-löslichen Polyestern verwendet werden können. Die Anyhdrid-Gruppierung stellt Carboxylgruppen für die Löslichkeit in Wasser zur Verfügung. Die Löslichkeit erzielt man durch Neutralisation mit Hilfe von Aminen, Metallhydroxiden oder Metallcarbonaten. Aus der DE-OS 26 37 167 und der US 3 523 998 ist bekannt, daß Polycarbonsäuren und ihre Anhydride in gleicher Weise wie Maleinsäureanhydrid und Trimellithsäureanhydrid zur Löslichmachung von Polyestern in Wasser beitragen. Für kosmetische Zwecke sind solche wasserlöslichen Polymeren bisher nicht bekannt.

Aus der DE-A 14 95 100 ist die Herstellung von Polyestern aus zur Anhydrierung befähigten mehrwertigen Carbonsäuren oder deren Anhydriden und mehrwertigen Alkoholen, gegebenenfalls unter Zusatz von Hydroxycarbonsäuren, bekannt. Diese Polyesterkomponenten werden zu Esterimidharzen, welche zur Lackherstellung dienen, weiter umgesetzt.

Die Erfindung betrifft nun die Verwendung von carboxylgruppenhaltigen Polykondensationsprodukten mit Glastemperaturen T_{G} ≧ 20°C aus Anhydriden von Tri- oder Tetracarbonsäuren und Diolen, Diaminen oder Aminoalkoholen für kosmetische Zwecke.

Tri- oder Tetracarbonsäuren, die zur Anhydridbildung befähigt sind, sind beispielsweise: 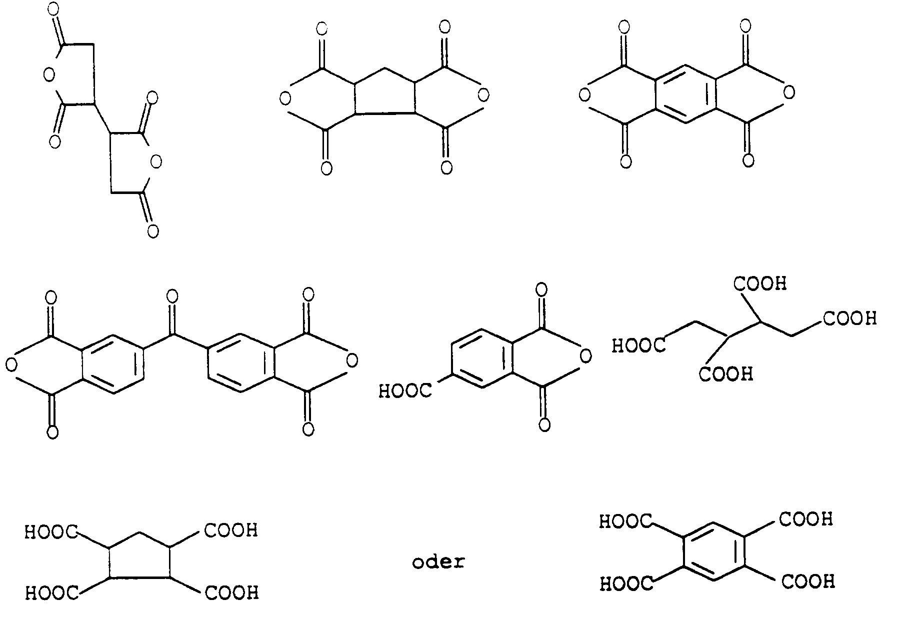
Bevorzugt sind dabei Pyromellithsäure und insbesondere deren Mono- oder Bisanhydrid sowie die Verbindungen der Formeln 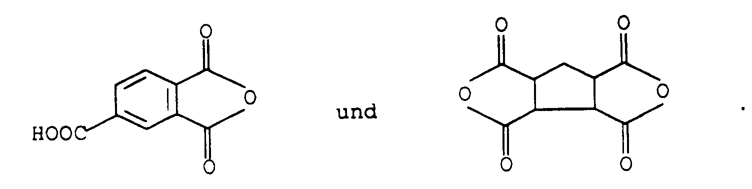
Diole, Diamine und Aminoalkohole für die Polykondensationsprodukte sind beispielsweise:
HOCH₂CH₂OH, CH₃CHOHCH₂OH, HO(CH₂)₄OH,
HO(CH₂)₆OH , 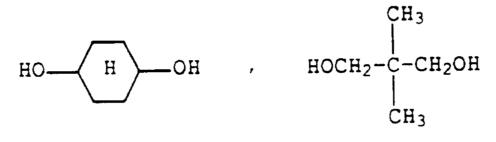 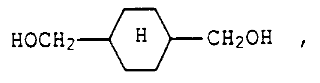
HO(CH₂CH₂O)ₙH oder HO[(CH₂)₄O]ₙH , wobei n 2 bis 50 ist, insbesondere kommen Verbindungen mit Molgewichten bis zu 2000 in Betracht. Ferner sind Polyesterole aus Phthal-, Isophthal- oder Terephthalsäure und Diolen mit Molgewichten bis zu 3000 zu nennen. Neben Phthalsäure können auch aliphatische Dicarbonsäuren, wie Adipinsäure oder Bernsteinsäure verwendet werden; insbesondere die Phthalsäuren können auch noch Substituenten wie Hydroxysulfonyl, vorzugsweise in Salzform (Li-, Na-, K- oder Ammoniumsalze) tragen.

Aminkomponenten haben beispielsweise die Formeln:
R¹HN-(CH₂)ₚNHR¹ mit p 2 bis 6, R¹ = C₁- bis C₄-Alkyl 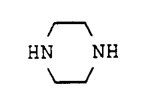
oder
HOCH₂CH₂NHR¹ .

Ein kleiner Teil der Diole, Diamine oder Aminoalkohole kann auch durch Triole oder Triamine ersetzt werden, insbesondere um durch Vernetzung höhere Molgewichte zu erzielen.

Bevorzugt von diesen Verbindungen sind z.B.:
HO(CH₂)ᵣOH, 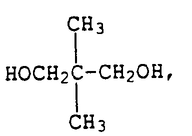
HOCH₂-CHOH-CH₃, 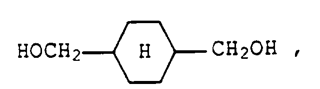
HO(C₂H₄O)₂H, 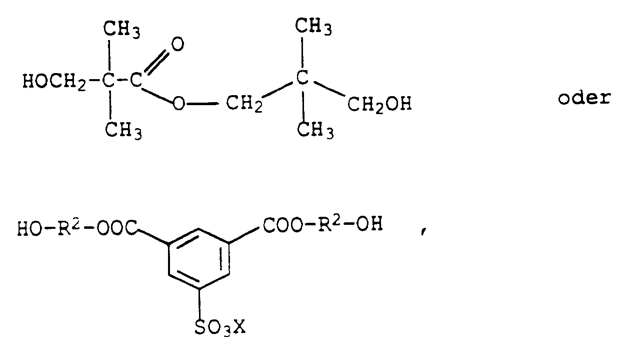
wobei r 2 bis 4 ist, X Wasserstoff oder ein Alkali- oder Ammoniumkation und R² C₂- bis C₈-Alkylen bedeuten.

Bevorzugt für R² sind z.B.
-CH₂CH₂-, 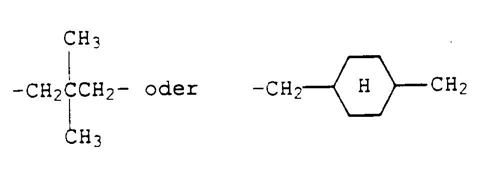
Die Polykondensationsprodukte werden vorzugsweise in ganz oder teilneutralisierter Form, z.B. als Alkali- oder insbesondere Salze von Aminen verwendet. Sie sollen Glastemperaturen von ≧ 20°C haben und sie sind in Abhängigkeit von der Zusammensetzung wasserlöslich oder wasserdispergierbar und löslich oder dispergierbar in niedermolekularen Alkoholen oder Ketonen.

Die Erfindung betrifft weiterhin als neue Verbindungen carboxylgruppenhaltige Polykondensationsprodukte enthaltend
a) Struktureinheiten der Formel 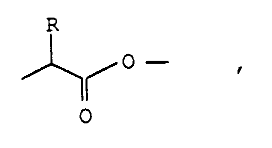
b) von zur Anhydridbildung befähigten Tri- oder Tetracarbonsäuren abgeleitete Reste und
c) von Diolen, Diaminen oder Aminoalkoholen abgeleitete Struktureinheiten, wobei
   - R: Wasserstoff, C₁- bis C₈-Alkyl oder Phenyl ist.

Alkylreste R sind z.B. C₂H₅, C₃H₇, C₄H₉, C₅H₁₁, C₆H₁₃, C₇H₁₅ oder C₈H₁₇, wobei n- oder i-Reste möglich sind, und vorzugsweise Methyl.

Die Struktureinheiten der Formel 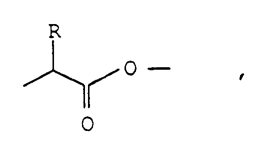
stammen von einer α-Hydroxycarbonsäure, wie α-Hydroxyessigsäure und insbesondere Milchsäure oder deren Derivaten (z.B. dem Lactid) . Wahrscheinlich enthalten die erfindungsgemäßen Produkte auch Blöcke der Formel 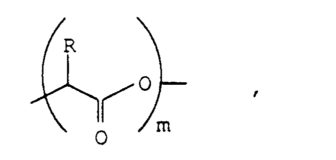
wobei m 1 bis 50, vorzugsweise 5 bis 20 sein kann.

Ein Teil der α-Hydroxycarbonsäure (0 bis 80 %, vorzugsweise 0 bis 30 %) kann auch durch eine andere Hydroxycarbonsäure ersetzt werden, insbesondere sind β-Hydroxycarbonsäuren wie β-Hydroxybuttersäure oder β-Hydroxyvaleriansäure oder auch Lactone wie ε-Caprolacton oder amingruppenhaltige Hydroxycarbonsäuren, z.B. das Umsetzungsprodukt aus Bernsteinsäureanhydrid und Methylethanolamin der Formel 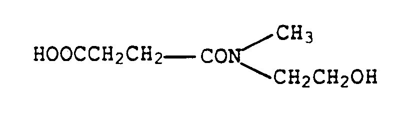
zu nennen.

Die Reste b) und c) stammen dabei von den bereits genannten Tri- und Tetracarbonsäuren bzw. den Diolen, Diaminen oder Aminoalkoholen.

Die erfindungsgemäßen Verbindungen sind in Abhängigkeit von der Zusammensetzung weich bis spröde, wasserlöslich oder wasserdispergierbar, löslich oder dispergierbar in niedermolekularen Alkoholen oder Ketonen und biologisch abbaubar, insbesondere wenn der Anteil an Struktureinheiten der Formel gemäß a) > 50 Gew.-% ist.

Sind die erfindungsgemäßen Verbindungen wasserdispergierbar, können sie in Form von wäßrigen Mikrodispersionen mit Teilchendurchmessern von üblicherweise 5 bis 100 nm, insbesondere 10 bis 80 nm, und Feststoffgehalten von üblicherweise 1 bis 40 Gew.-%, insbesondere 3 bis 30 Gew.-%, zur Anwendung gebracht werden. Diese Mikrodispersionen benötigen in der Regel keine Emulgatoren oder Tenside zu ihrer Stabilisierung.

Insbesondere betrifft die Erfindung Polykondensationsprodukte, die
von 30 bis 87 Gew.-% an a),
von 10 bis 32 Gew.-% an b) und
von 3 bis 48 Gew.-% an c)
enthalten und die sich als Haarbehandlungsmittel eignen.

Bevorzugt sind für diesen Zweck Produkte mit Gehalten an
a) von 40 bis 80 Gew.-%,
b) von 16 bis 30 Gew.-% und
c) von 4 bis 30 Gew.-%.

Zur Herstellung der erfindungsgemäßen Polykondensationsprodukte geht man zweckmäßigerweise so vor, daß man die Ausgangsverbindungen für a), b) und c) mischt und unter Inertgas-Atmosphäre auf Temperaturen von ungefähr 110 bis 240°C, vorzugsweise 130 bis 200°C, erhitzt und das dabei entstehende Wasser abdestilliert. Der Zusatz von üblichen Veresterungskatalysatoren, wie Tetraisopropyltitanat (in üblichen Mengen, z.B. 10 bis 50 ppm) ist dabei zweckmäßig.

Man kann die Reaktion auch in Gegenwart von inerten organischen Lösemitteln beginnen, die im Laufe der Reaktion wieder abdestilliert werden. Solche Lösungsmittel sind z.B. Wasser, Tetrahydrofuran oder Ethylenglykoldimethylether. Es wird dabei soviel Lösungsmittel abdestilliert, daß die Reaktionsmischung immer klar bleibt. Nach der Reaktion, d.h. wenn die Säurezahl des Produktes den gewünschten Wert erreicht, wird das Reaktionsgemisch ungefähr 3 bis 5 h bei einer Temperatur von 150 bis 200°C unter vermindertem Druck erhitzt.

Einzelheiten der Reaktionsführung können den Beispielen entnommen werden, in denen sich Angaben über Teile und Prozente, sofern nicht anders vermerkt, auf das Gewicht beziehen.

Die erfindungsgemäßen Produkte haben in der Regel K-Werte von 10 bis 90, vorzugsweise 20 bis 50, gemessen nach Fikentscher (1 %ige NMP-Lösung, 25°C, pH 7) und Säurezahlen von 10 bis 200, bevorzugt 40 bis 150.

Für die Verwendung als Haarbehandlungsmittel sollten die Carboxylgruppen der erfindungsgemäßen Produkte vorzugsweise in Salzform vorliegen, wobei Natrium und Ammoniumionen abgeleitet von den Verbindungen 2-Amino-2-methylpropanol, Diethylaminopropylamin, Triisopropanolamin, Methyldiethanolamin, Dimethylethanolamin und Imidazol bevorzugt sind.

### Beispiele

Allgemeine Vorschrift zur Herstellung von wasserlöslichen Polyestern:
In einem mit Rührer, Innenthermometer, Gaseinleitungsrohr und absteigendem Kühler versehenen Vierhalskolben werden Milchsäure (90 %ige Lösung), Diol (bzw. Diamin oder Aminoalkohol) und Anhydrid zusammen unter Stickstoff und Rühren auf 150°C erhitzt, wobei zunächst das Wasser bei ungefähr 100°C aus der Milchsäurelösung abdestilliert wird. Die Reaktionstemperatur wird etwa 3 h bei 150° gehalten, dann wird sie stündlich um 10°C gesteigert, bis sie etwa 200 ± 20°C erreicht. Das Reaktionswasser wird dabei solange abdestilliert, bis ungefähr die theoretische Säurezahl erreicht ist. Anschließend erhitzt man, eventuell auch unter Zugabe von Katalysator, noch 2 - 4 Stunden unter einem vermindertem Druck von ungefähr 10 mmHg. Man erhält nach der Abkühlung auf Raumtemperatur ein klares, hellgelbes bis gelbes Produkt, welches nach der Neutralisation mit 2-Amino-2-methylpropanol in Wasser und in Ethanol gut löslich oder dispergierbar ist.

Es werden in den Beispielen (die Produkte wurden nach der allgemeinen Vorschrift hergestellt) folgende Abkürzungen verwendet.
- MIS: = 90 %ige wäßrige Lösung von Milchsäure
- EG: = Ethylenglykol
- TMP: = Trimethylolpropan
- PMDA: = Pyromellithsäuredianhydrid
- P(IPS:NPG): = Polyesterdiol M_{w} = 800 g/mol wurde aus Isophthalsäure und Neopentylglykol hergestellt.
- P(SIPS:NPG): = hat die Struktur 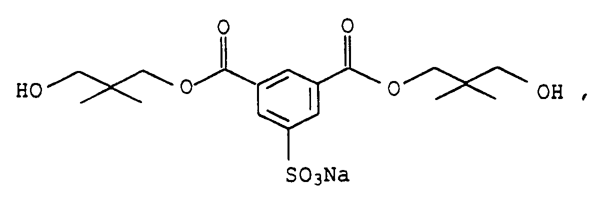 es wurde aus 5-Natriumsulfonato-isophthalsäure und Neopentylglykol hergestellt.

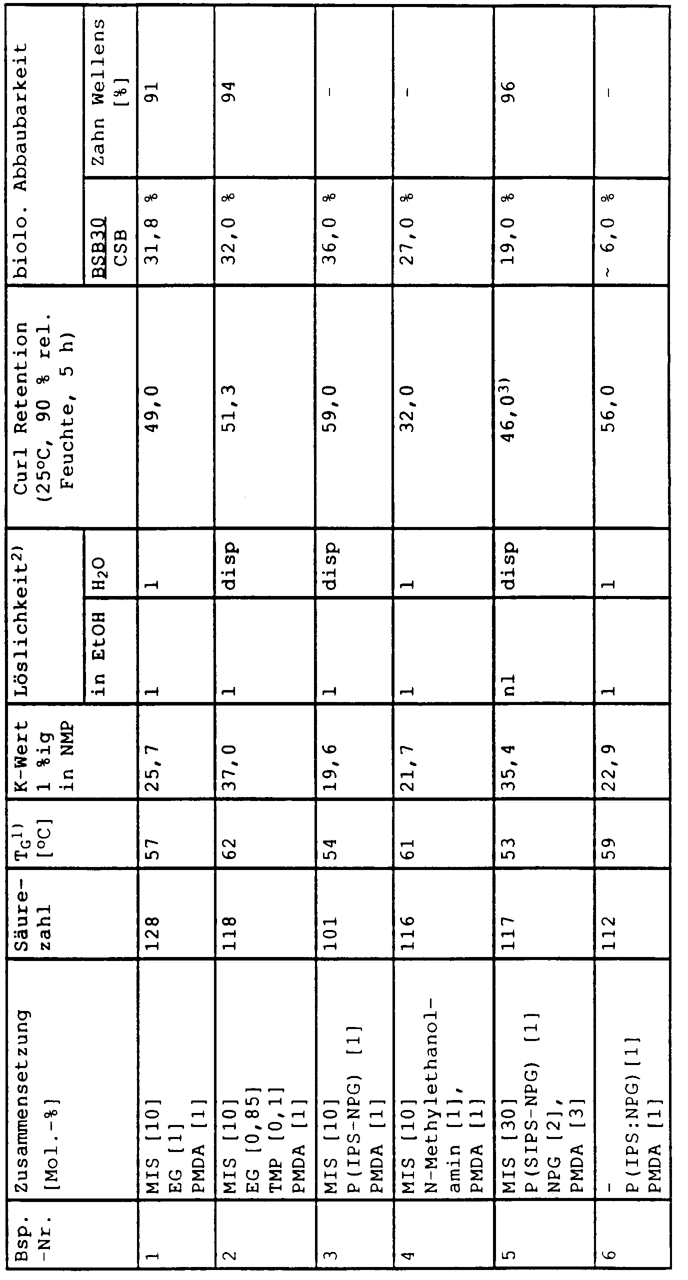

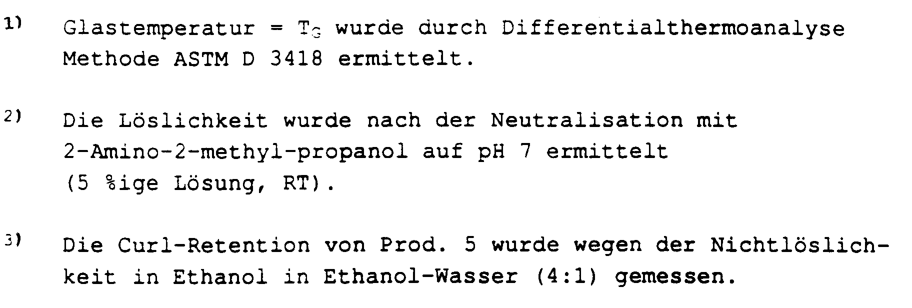

### Weitere angewendete Prüfmethode:

1. Curl-Retention:
   Die Curl-Retention ist ein Maß für die Haarfestigungswirkung. Sie wird im Modellversuch gemessen an Haarlocken, erzeugt durch eine übliche Wasserwelle an ca. 15 cm langen Haaren, die mit 2 gew.-%iger Spraylösung eines Harzes gemäß Tabelle 1 oder 2 und zu 75 % teilneutralisiert aus 10 cm Entfernung 4 sec lang besprüht werden. Nach 5-stündiger Behandlung der aufgehängten Locken in einer Klimakammer (25°C, 90 % relative Luftfeuchte) wird die relative Verformung (Aufweitung) der Locken, bezogen auf die ursprüngliche Form, festgestellt. Ein hoher Wert bedeutet hohe Festigungswirkung, d.h. 100 % wäre Erhalt der ursprünglichen Form der aufgehängten Locke, 0 % wäre ein völlig gestrecktes Haar.
2. Biologische Abbaubarkeit
   2.1 BSB30/CSB
      - BSB:: Methoden zur Bestimmung des Biochemischen Sauerstoffbedarf nach Analysenvorschrift: DIN 38409 Teil 51
      - BSB 30:: BSB nach 30 Tagen.
      - CSB:: Methode zur Bestimmung des Chemischen Sauerstoffbedarf nach Analysenvorschrift: DIN 38409 Teil 41.
   2.2 Standversuch nach Zahn-Wellens: nach 28 Tagen
      Analysen Vorschrift: DIN 38412, Teil 25.

### Anwendungsbeispiele

| 1. Aerosol-Haarspray | |
|---|---|
| Polymeres gemäß Beispiel 2 | 3,00 % |
| 2-Amino-2-methyl-propanol | 0,64 % |
| Ethanol | 51,36 % |
| Wasser dest. | 10,00 % |
| Dimethylether | 35,00 % |
| Parfümöl | q.s. |

| 2. Handpumpen-Haarspray | |
|---|---|
| Polymeres gemäß Beispiel 2 | 3,00 % |
| 2-Amino-2-methyl-propanol | 0,64 % |
| Ethanol | 20,45 % |
| Wasser dest. | 40,91 % |
| Parfümöl | q.s. |

| 3. Haarfestiger | |
|---|---|
| Polymeres gemäß Beispiel 1 | 4,00 % |
| 2-Amino-2-methyl-propanol | 0,82 % |
| Wasser dest. | 95,18 % |
| Parfümöl | q.s. |

| 4. Haarfestiger | |
|---|---|
| Polymeres gemäß Beispiel 1 | 4,00 % |
| 2-Amino-2-methyl-propanol | 0,82 % |
| Ethanol | 31,73 % |
| Wasser dest. | 63,45 % |
| Parfümöl | q.s. |

## Patentansprüche

1. Carboxylgruppenhaltige Polykondensationsprodukte, enthaltend
a) Struktureinheiten der Formel 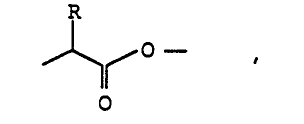
b) von zur Anhydridbildung befähigten Tri- oder Tetracarbonsäuren abgeleitete Reste und
c) von Diolen, Diaminen oder Aminoalkoholen abgeleitete Struktureinheiten, wobei
R Wasserstoff, C₁- bis C₈-Alkyl oder Phenyl ist.

2. Polykondensationsprodukte gemäß Anspruch 1, enthaltend als Komponente b) Pyromellithsäure, deren Mono- oder Dianhydrid oder die Verbindungen der Formeln

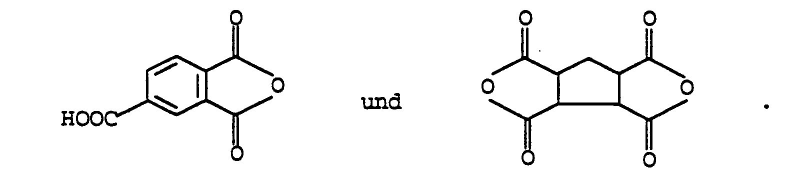

3. Polykondensationsprodukte gemäß Anspruch 1, die
von 30 bis 87 Gew.-% an a),
von 10 bis 32 Gew.-% an b) und
von 3 bis 48 Gew.-% an c)
enthalten.

4. Produkte gemäß Anspruch 1 mit Gehalten an
a) von 40 bis 80 Gew.-%,
b) von 16 bis 30 Gew.-% und
c) von 4 bis 30 Gew.-%.

5. Verwendung der Polykondensationsprodukte gemäß den Ansprüchen 1 bis 4 als Haarbehandlungsmittel.

## Claims

1. A carboxyl-containing polycondensation product comprising
a) structural units of the formula 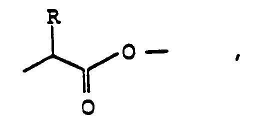
b) radicals derived from tricarboxylic or tetracarboxylic acids capable of anhydride formation and
c) structural units derived from diols, diamines or amino alcohols, where
R is hydrogen, C₁- to C₈-alkyl or phenyl.

2. A polycondensation product as claimed in claim 1, containing as component b) pyromellitic acid, the monoanhydride or dianhydride thereof or the compounds of the formulae

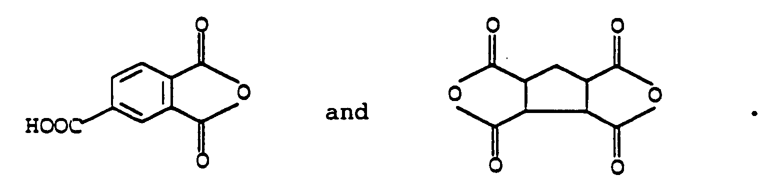

3. A polycondensation product as claimed in claim 1, which contains
from 30 to 87% by weight of a),
from 10 to 32% by weight of b) and
from 3 to 48% by weight of c).

4. A product as claimed in claim 1, containing
from 40 to 80% by weight of a),
from 16 to 30% by weight of b) and
from 4 to 30% by weight of c).

5. The use of a polycondensation product as claimed in any of claims 1 to 4 as a hair treatment composition.

## Revendications

1. Produits de polycondensation à groupes carboxyle contenant :
a) des unités de structure de formule 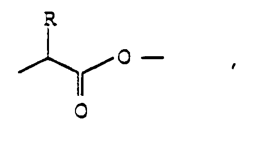
b) des restes dérivés d'acides tri- ou tétracarboxyliques aptes à la formation d'anhydride et
c) des unités de structure, dérivés de diole, diamines ou aminoalcools,
R étant hydrogène, alkyle en C1 à C8 ou phényle.

2. Produits de polycondensation selon la revendication 1, contenant comme composés b) des acides pyromellitiques, leur mono- ou dianhydride ou les composés des formules

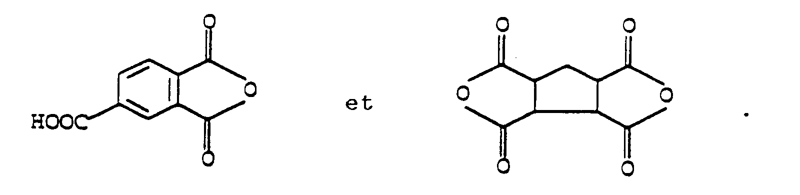

3. Produits de polycondensation selon la revendication 1, qui contiennent
de 30 à 87 % en poids de a)
de 10 à 32 % en poids de b) et
de 3 à 48 % en poids de c).

4. Produits de polycondensation selon la revendication 1, à teneurs en
a) de 40 à 80 % en poids
b) de 16 à 30 % en poids et
c) de 4 à 30 % en poids.

5. Utilisation des produits de polycondensation selon les revendications 1 à 4, comme agents de traitement des cheveux.
